(19) **Europäisches Patentamt / European Patent Office / Office européen des brevets**

(11) **EP 2 514 815 B1**

(12) **EUROPEAN PATENT SPECIFICATION**

(45) Date of publication and mention of the grant of the patent:
**02.10.2019 Bulletin 2019/40**

(21) Application number: **09850753.6**

(22) Date of filing: **23.11.2009**

(51) Int Cl.:
*C12N 1/14* (2006.01)     *A01P 3/00* (2006.01)
*C12R 1/885* (2006.01)     *C05F 11/08* (2006.01)

(86) International application number:
**PCT/CN2009/075076**

(87) International publication number:
**WO 2011/050547 (05.05.2011 Gazette 2011/18)**

(54) **BIOCONTROL STRAIN FOR CUCUMBER AND WATERMELON CONTINUOUS CROPPING WILT DISEASES AND MICROBIAL ORGANIC FERTILIZER THEREOF**

STAMM ZUR BIOLOGISCHEN BEKÄMPFUNG DER WELKEKRANKHEIT BEI DAUERKULTUREN VON GURKEN UND WASSERMELONEN SOWIE MIKROBIELLE ORGANISCHE DÜNGER DAFÜR

SOUCHE UTILISÉE POUR LA LUTTE BIOLOGIQUE CONTRE LA FLÉTRISSURE BACTÉRIENNE DU CONCOMBRE ET DU MELON D'EAU EN CULTURE CONTINUE ET ENGRAIS ORGANIQUE MICROBIEN À BASE DE CELLE-CI

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **29.10.2009 CN 200910233576**

(43) Date of publication of application:
**24.10.2012 Bulletin 2012/43**

(73) Proprietors:
• **Nanjing Agricultural University**
  **Jiangsu 210095 (CN)**
• **Jiangsu New Ground Bio-Fertilizer Engineering Center Co., Ltd**
  **Yixing, Jiangsu 214200 (CN)**

(72) Inventors:
• **SHEN, Qirong**
  **Nanjing**
  **Jiangsu 210095 (CN)**
• **CHEN, Lihua**
  **Nanjing**
  **Jiangsu 210095 (CN)**
• **YANG, Xingming**
  **Nanjing**
  **Jiangsu 210095 (CN)**
• **XU, Yangchun**
  **Nanjing**
  **Jiangsu 210095 (CN)**

• **HUANG, Qiwei**
  **Nanjing**
  **Jiangsu 210095 (CN)**
• **SHEN, Biao**
  **Nanjing**
  **Jiangsu 210095 (CN)**
• **RAN, Wei**
  **Nanjing**
  **Jiangsu 210095 (CN)**

(74) Representative: **Rippel, Hans Christoph et al**
**Patentanwälte**
**Isenbruck Bösl Hörschler PartG mbB**
**Eastsite One**
**Seckenheimer Landstrasse 4**
**68163 Mannheim (DE)**

(56) References cited:
**WO-A1-2009/083819      WO-A2-2007/110686**
**CN-A- 101 057 593      CN-A- 101 485 336**
**CN-A- 101 503 659      CN-A- 101 575 574**
**CN-C- 1 236 051      CN-C- 100 500 005**
**US-A1- 2006 292 124**

- DATABASE BIOSIS [Online] BIOSCIENCES INFORMATION SERVICE, PHILADELPHIA, PA, US; 5 June 1990 (1990-06-05), CHO C T ET AL: "BIOLOGICAL CONTROL OF FUSARIUM-OXYSPORUM-F-SP-CUCUMERINUM CAUSING CUCUMBER WILT BY GLIOCLADIUM-VIRENS AND TRICHODERMA-HARZIANUM", XP002328036, retrieved from BIOSIS Database accession no. 199099090007953
- LIN WEI ET AL: "Test on the control efficacy of antagonistic microorganism on watermelon wilt disease", JOURNAL OF GUANGXI AGRIC AND BIOL SCIENCE,, vol. 21, no. 4, 31 December 2002 (2002-12-31), pages 242-244, XP008168460,
- PATIBANDA A K ET AL: "Efficacy of Trichoderma harzianum Rifai alone or in combination with fungicides against Sclerotium wilt of groundnut", JOURNAL OF BIOLOGICAL CONTROL, INDIAN SOCIETY FOR BIOCONTROL ADVANCEMENT, COIMBATORE, IN, vol. 16, no. 1, 1 January 2002 (2002-01-01), pages 57-63, XP009175123, ISSN: 0970-5732
- LIN WEI ET AL.: 'Test on the control efficacy of antagonistic microorganism on watermelon wilt disease' JOURNAL OF GUANGXI AGRIC AND BIOL SCIENCE vol. 21, no. 4, 31 December 2002, XP008168460

**Description**

**Field of the Invention**

The present invention relates to a biocontrol strain against the wilt of continuous cropping cucumber and watermelon as well as its microbial organic fertilizer, which belongs to the technology for intensive agricultural production and is dedicated to overcoming and eliminating the wilt of continuous cropping cucumber and watermelon.

**Background of the Invention**

Cucumber and watermelon are important economic crops in China, Their yield and quality are related to the national economy and the people's livelihood. Cucumber and watermelon wilt is one of the major diseases of cucumber and watermelon and particularly does serious harm on the planting in protected land. Cucumber wilt was first reported in the Netherlands in 1943. In 1949, it destroyed above 40% of the cucumber in Florida, the USA and infected more than 70% of the continuous cropping plants in the third season. Currently, in the highly intensive cucumber and watermelon growing areas in China, a serious wilt can result in loss of about 90% of yield. Cucumber and watermelon wilt is a soilborne fungal disease. It is widely distributed in the tropical zone, subtropical zone and some warm areas in the world. *Fusarium oxysporum* - the pathogen of cucumber and watermelon wilt can be spread through soil, flowing water (for example, irrigation), seeds and other means and is highly adaptable to the environment. Once cucumber is infected with *Fusarium oxysporum,* it will damp off before or after it comes out of the soil, the vine and stem at the top will wither, the vascular bundles at bud base will die and in the end the entire plant will perish. *Fusarium oxysporum* can result in the damping-off, cortex decay and plant dwarfing of watermelon in seedling stage, and the sudden or gradual wilting of the old plants. Under the condition of high temperature and high humidity, the disease develops rapidly, and the morbidity in the fields with serious attack of wilt is 100%, resulting in no harvest.

On the other hand, the straw of paddy, wheat, corn, rape and other crops is burned on the spot and the excrement of the livestock and poultry raised on a scale is discarded. It does not only seriously pollute environment but also is a great waste of the raw material that can be used to produce organic fertilizer and bio-organic fertilizer products; enormous nutritive resources (C, N, P, K, S and trace elements) are lost outside the soil - plant system and the capability of sustainable development of Chinese agriculture is obviously weakened. How to maximally return the nutritive elements taken away from soil due to harvesting of crops to the soil? The only way is to make these solid organic wastes into commercial organic fertilizer and apply the fertilizer to the soil. If these solid organic wastes are synthesized into high-grade organic compost through high-temperature fermentation and then the compost is used as a carrier of functional bacteria to prepare microbial organic fertilizer, the function will be clear and there will be a good application prospects. Cho et al.: "BIOLOGICAL COONTROL OF FUSARIUM-OXYSPRORUM-F-SP-CUCUMBERING CAUSING CUCUMBER WILT BY GLIOCLADIUM-VIRENS AND TRICHODERMA-VIRENS" (Biosis, database accession no. 1900000753, Patent application CN 101 057 53 A and LIN WEI et al. : "Test on the control efficacy of antagonistic microorganism on watermelon wilt disease", JOURNAL OF GUANGXI AGRIC AND BIOL SCIENCE, vol. 21, no. 4, 31 December 2002, pages 242-244 disclose Trichoderma Harzianum strains for preventing or eliminating cucumber or watermelon wilt.

**Summary of the Invention**

**Technical Problem**

The purpose of the present invention is to develop an antagonistic microbial organic fertilizer that can prevent and eliminate cucumber and watermelon wilt. The controlling rate of this fertilizer in fields is above 80%. It can biologically restore the soil with continuous cropping obstacle and ensure the smooth development of intensive agriculture.

**Technical Solution**

The biocontrol strain SQR-T037 that is used to prevent and eliminate the wilt of continuous cropping cucumber and watermelon belongs to *Trichoderma harzianum.* It was collected by China General Microbiological Culture Collection Center (CGMCC) on September 22, 2009 , and the culture collection number is CGMCC No.3308. The main biological characteristics are follows: the optimum growth temperature on potato-dextrose-agar (PDA) culture medium is 30°C; the diameter of colony can be reached 90mm and it can cover the whole dish and generate yellow pigment in the culture medium when incubated for 96 hours; the conidiophore is in a shape of bottle; the conidium is oval, 2.3-3.5$\mu$m long and 2.0-3.2$\mu$m wide, its surface is smooth. At the beginning color of conidium is yellow green, then it changes into dark green slowly.

In the microbial organic fertilizer produced from the above biocontrol strain that prevents and eliminates the wilt of continuous cropping cucumber and watermelon, the content of SQR-T037 strain is above $1\times10^8$cfu/g, the content of total nitrogen is 4~5% (weight percent), above 90% of the total nitrogen is organic nitrogen, the content of total nitrogen-phosphorus-potassium nutrient is 6~10% (weight percent) and the content of organic matter is 30~35% (weight percent).

The microbial organic fertilizer that prevents and eliminates the wilt of continuous cropping cucumber and watermelon is produced by the following method:

1) SQR-T037 is incubated in PDA culture to conduct liquid fermentation production under the following conditions: the initial pH range for fermentation is 6.5-7.2; the culture temperature is 25-30°C; DO(dissolved oxygen): ventilation range is 30~100%, 170rpm; the fermentation time is 48h; all mycelium pellets are broken into colony formation units of this strain in the late stage of fermentation, and the colony count of SQR-T037 in fermentation liquor is $\geq 1 \times 10^9$cfu/mL; the preparation method of the used PDA culture (taking the preparation of 1L of culture medium for example): Unpeel 200g of potato, cut it into small dice, and boil it in boiling water for 30min, then filter the mixture, add 20g of common sucrose into the filtrate, fix the volume at 1000 mL, adjust pH value to 6.5-7.0, and sterilize the liquor at 121°C for 20min.

2) SQR-T037 fermentation liquor is respectively incubated to the mature livestock or poultry excrement compost and the mixture from microbial enzymatic hydrolysis of rapeseed meal at a dosage of 50L/t to conduct solid fermentation. The fermentation temperature is 30-50°C. During the fermentation, the material is turned over once a day. The fermentation is completed in 5-7 days to ensure the colony count of this strain is above $1\times10^8$ cfu/g. In the end, the solid microbial agent of biocontrol strain SQR-T037 is obtained; the germination index of the mature livestock or poultry excrement compost is more than 98%; the content of organic matter is $\geq 35\%$; the content of organic nitrogen is 1.2-2% and water content is 25-30%.

3) The solid microbial agent SQR-T037 made from livestock or poultry excrement compost (50-80% v/v) and the solid microbial agent SQR-T037 made from the mixture from enzymatic hydrolysis of rapeseed meal (20-50% v/v) are thoroughly mixed. The mixture is ripened 2-3 days. During the ripening, it is turned over twice. In the end, the microbial organic fertilizer is evaporated at temperature of below 50°C till its water content is less than 30%. After packaging, the microbial organic fertilizer that prevents and eliminates the wilt of continuous cropping cucumber and watermelon is obtained.

The microbial organic fertilizer can be exclusively used to prevent and eliminate the wilt of continuous cropping cucumber and watermelon.

### Beneficial Effects

The present invention relates to a microbial organic fertilizer that can overcome or eliminate the wilt of continuous cropping cucumber and watermelon as well as its producing method. The livestock or poultry excrement compost and the amino acid mixture from enzymatic hydrolysis of rapeseed meal are mixed with the biocontrol strain liquid to produce microbial organic fertilizer. Comparing with the products in the current market, the product has the following advantages:

1) This fertilizer product contains high-performance strain (*Trichoderma harzianum*) that inhibits the growth of the pathogenic fungi of cucumber and watermelon wilt. The inhibition effect is very remarkable. The experiment result indicates that after this product is applied to the soil suffering from the wilt of continuous cropping cucumber and watermelon, the biocontrol rate of cucumber and watermelon wilt is above 85%, and the biocontrol rate of the soil applied with this fertilizer in three consecutive years is even higher.

2) This fertilizer is bio-organic fertilizer and contains rich organic matter (the content is 30-35%) and organic nitrogen (the content is 3-5%). The content of total phosphor in this product is 2-4%. Moreover, this product has brilliant bioavailability. After this product is used, crops can smoothly get through phosphorin sensitive period in seedling stage. In comparison, after phosphoric fertilizer is applied to soil, the bioavailability is poor. The rich nutrition in bio-organic fertilizer also provides a condition for the growth and multiplication of the biocontrol strain in the fertilizer so that it can survive in soil and form a dominant microflora, thus playing a role in preventing disease.

3) As it is a bio-strain preparation, it doesn't have any problem caused by the use of chemical pesticides and is conducive to the pollution-free production of cucumber and watermelon. Farmers need not use other chemical pesticides that prevent and cure wilt or can reduce the dosage of other chemical pesticides. This not only can reduce farmers' expenditure but also can improve the quality of agricultural products. Meanwhile, this microbial fertilizer has the function of increasing yield, so farmers' revenue can be increased.

**Brief Description of the Drawings**

Fig. 1: Effect of biocontrol strain SQR-T037 on inhibiting the pathogen of cucumber and watermelon wilt

A: Effect drawing of the inhibition of SQR-T037 to the pathogen of cucumber wilt

B: Effect drawing of the inhibition of SQR-T037 to the pathogen of watermelon wilt

Fig. 2:Controlling effect in greenhouse pot experiment

A: Effect of the bio-organic fertilizer made from SQR-T037 on inhibiting cucumber wilt $CK_1$: Control; $CK_2$: Soil for continuous cropping of cucumber; $T_1$: $CK_2$ + organic fertilizer; $T_2$: $CK_2$ + fermentation liquor of *Trichoderma* SQR-T037; $T_3$: $CK_2$ + mixed organic fertilizer of *Trichoderma* SQR-T037; T4: $CK_2$+ organic fertilizer generated from fermentation of *Trichoderma* SQR-T037

B: Effect of the bio-organic fertilizer made from SQR-T037 on inhibiting watermelon wilt $CK_1$: Control; $CK_2$: Soil for continuous cropping of watermelon; $T_1$: $CK_2$ + organic fertilizer; $T_2$: $CK_2$ + Organic fertilizer generated from fermentation of *Trichoderma* SQR-T037

**Detailed Description of the Embodiments:**

(I) Strain separation and identification

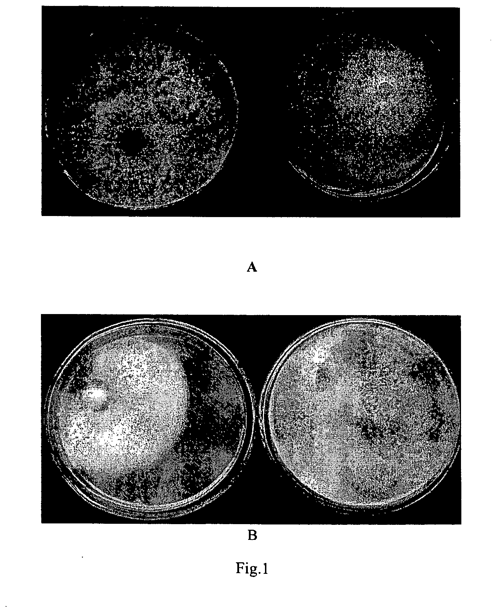

Samples of the plants with continuous cropping obstacle and serious symptoms of wilt as well as the surrounding soil are collected and stored at low temperature. The selective medium of *Fusarium oxysporum* is adopted to separate pathogens - *Fusarium oxysporum* f. sp. *Cucumerinum* and *Fusarium oxysporum* f. sp. *niveum.* Then the separated pathogenic bacteria of wilt are used as indicators, and the selective medium of *Tri -choderma* is used to separate biocontrol strain from the organic compost that has been composted at high temperature. In the end, through pot experiment and field experiment, high-performance biocontrol strain SQR-T037 is obtained by means of secondary screening. The strain is identified. It is stored in PDA culture media at 4°C and at room temperature. Biocontrol strain SQR-T037 can fully inhibit the growth of pathogens in 72h (Fig. 1).

Strain SQR-T037 belongs to *Trichoderma harzianum.* The main biological characteristics are: the optimum growth temperature on PDA culture medium is 30°C;the diameter of colony can be reached 90mm and it will cover the whole dish and generate yellow pigment in the culture medium when incubated for 96 hours; the conidiophore is in a shape of bottle; the conidium is oval, 2.3-3.5$\mu$m long and 2.0-3.2$\mu$m wide, and its surface is smooth; at the beginning its color is yellow green, then it changed into dark green slowly.

(II) Production of the bacterial preparation

1) SQR-T037 is incubated to PDA culture to conduct liquid fermentation production under the following conditions: the initial pH range is 6.5-7.0; the culture temperature is 25°C; DO: ventilation range is 30~100% (v/v), 170rpm; the fermentation time is 48h; all mycelium pellets are broken into colony formation unit of this strain in the late stage of fermentation, and the colony count (mycelium fragment) of SQR-T037 in fermentation liquor is $\geq 1\times 10^9$cfu/mL; Preparation method of the used PDA culture (taking the preparation of 1L of culture medium for example): Unpeel 200g of potato, cut it into small dice, and boil it in boiling water for 30min, tben filter the mixture, add 20g of common sucrose into the filtrate, fix the volume at 1000 mL, adjust pH value to 6.5-7.0, and sterilize the liquor at 121°C for 20min.

2) SQR-T037 fermentation liquor is incubated to the mature pig excrement compost and the mixture from microbial enzymatic hydrolysis of rapeseed meal at a dosage of 50L/t to conduct solid fermentation. During the fermentation, the material is turned over once a day to ensure the fermentation temperature is 30-50°C. The fermentation is completed in 5-7 days to ensure the colony count of the biocontrol strain is above $1\times 10^8$ cfu/g. In the end, the solid microbial agent of biocontrol strain SQR-T037 is obtained; the germination index of the mature pig excrement compost is more than 98%, the content of organic matter is $\geq$35%, the content of organic nitrogen is 1.2-2% and water content is 25-30%.

The mixture from microbial enzymatic hydrolysis of rapeseed meal is produced by the following method (known and used by the public, see Chinese invention patent ZL200610086 126.0, a biological preparation method of amino acids for agricultural use and their fertilizer product): add the fermentation liquor of *Steno trophomonas maltophilia* strain 37-1 into raw material rapeseed meal; adjust water content to 55-65% and pH value to 6.0-7.5; conduct open solid fermentation; turn over it once the fermentation temperature rises to 50°C, turn it over every day since then, and maintain the temperature at 35-50°C for about 5-7 days. After solid fermentation starts, the pH value of the material will keep rising. Whenever it is turned over, acidic liquid should be added to adjust water content and pH value and maintain water content at 55-65% and pH value at 6.0-7.0. When fermentation is terminated, acidic liquid will be sprayed again till pH value of the material is about 5.0. After that, the material is dried at low temperature or by air. The final product is the mixture from microbial enzymatic hydrolysis of rapeseed meal (mixture containing amino acids).

3) The solid microbial agent SQR-T037 made from pig excrement compost (80% v/v) and the solid microbial agent SQR-T037 made from the mixture from microbial enzymatic hydrolysis of rapeseed meal (20% v/v) are thoroughly mixed. The mixture is ripened 2-3 days. During the ripening, it is turned over twice. In the end, the microbial organic fertilizer is evaporated at temperature of below 50°C till its water content is less than 30%. Thus the microbial organic fertilizer that controls the wilt of continuous cropping cucumber and watermelon is obtained.

In the microbial organic fertilizer (microbial organic fertilizer that prevents and eliminates the wilt of continuous cropping cucumber and watermelon) generated from the re-fermentation of the above SQR-T037, the colony count of the biocontrol strain SQR-T037 is above $1 \times 10^8$cfu/g, the content of total nitrogen is 4~5% (weight percent), above 90% of the total nitrogen is organic nitrogen, total nitrogen-phosphorus-potassium nutrient is 6~10% (weight percent) and organic matter is 30~35% (weight percent).

(III) Greenhouse pot experiment

1. Wilt prevention and cure in the soil for continuous cropping of cucumber

The experimental soil is continuous cropping soil, with serious wilt. The extensively planted "Jin Chun 4" cucumber is adopted. The treatment is as follows:

$CK_1$: Control; $CK_2$: Soil for continuous cropping of cucumber; $T_1$: $CK_2$ + organic fertilizer; $T_2$: $CK_2$ + fermentation liquor of SQR-T037; $T_3$: Microbial organic fertilizer produced through simply mixing $CK_2$+SQR-T037 and organic fertilizer; $T_4$: Microbial organic fertilizer produced through re-fermentation of $CK_2$+SQR-T037 and organic fertilizer

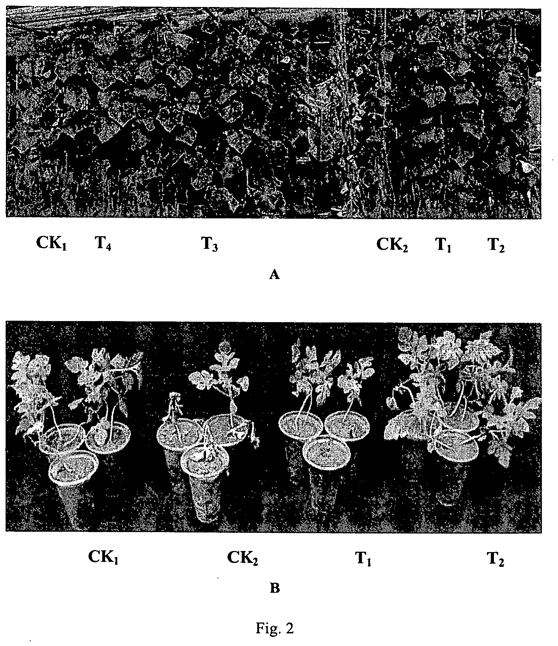

Each treatment is repeated five times, and 10kg of sick soil is used in per pot. $T_2$: Apply 10mL of SQR-T037 fermentation liquor in each pot; $T_3$: Apply 20g of microbial organic fertilizer containing SQR-T037 (simple mixture of 10mL of SQR-T037 fermentation liquor and 20g of organic fertilizer); $T_4$: Apply 20g of microbial organic fertilizer generated from fermentation of SQR-T037. The materials are mixed well to ensure the concentration of the biocontrol strain in soil reaches $10^5$cfu/g of dry soil. Five days after cucumber seedlings are transplanted, the morbidity is recorded. After 40 days, morbidity of $CK_2$ reaches 98.9%, whereas the incubation of SQR-T037 fermentation liquor, the microbial organic fertilizer containing SQR-T037 and the microbial organic fertilizer generated from fermentation of SQR-T037 all can effectively reduce the morbidity of wilt. $T_4$: The microbial organic fertilizer generated from fermentation of $CK_2$+SQR-T037 has the best control effect (Table 1, Fig. 2 A) and its morbidity is 7.3%.

2. Wilt control in the soil for continuous cropping of watermelon

The experimental soil is continuous cropping soil, with serious wilt. The treatment is as follows:

$CK_1$: Control; $CK_2$: Soil for continuous cropping of watermelon; $T_1$: $CK_2$+ organic fertilizer; $T_2$: Microbial organic fertilizer produced through re-fermentation of $CK_2$+SQR-T037 and organic fertilizer;

Each treatment is repeated ten times. Each repetition uses 300g of sick soil/cup. $T_1$: 3g of ordinary organic fertilizer is applied in each cup; $T_2$: Apply 3g of the organic fertilizer generated from re-fermentation of SQR-T037. The materials are mixed well. The concentration of SQR-T037 in soil is $10^5$cfu/g of dry soil. Five days after watermelon seedlings are transplanted, the morbidity is recorded. After 21 days, the morbidity of $CK_2$ reaches 95.38%, whereas $T_2$: the microbial organic fertilizer generated from re-fermentation of $CK_2$+SQR-T037 can effectively reduce wilt morbidity and control it at 2.6%. (Table 2 and Fig. 2B)

The results indicate that the application of biocontrol strain SQR-T037 can not only control cucumber and watermelon wilt, greatly reduce the quantity of wilt pathogens (F. *oxysporum*) in soil, promote the changes of the microflora at rhizosphere of cucumber and watermelon, remarkably increase beneficial bacterial flora and remarkably reduce the total quantity of fungi and the quantity of pathogens (Table3), but also can remarkably increase the chlorophyll content, root system activity and solid content of cucumber and watermelon (Table 1 and Table 2).

After biocontrol strain is applied, the activity of the enzymes relating to systematic disease resistance of cucumber and watermelon is remarkably increased (Table4).

(IV) Field experiment

SQR-T037 organic fertilizer was applied in the field that was infected by cucumber wilt in Yixing, Jiangsu in 2008.
Treatment of field experiment: 1. Apply organic fertilizer; 2. Apply the microbial organic fertilizer formed from the re-fermentation of SQR-T037 and organic fertilizer. The field with serious infection of wilt is randomly divided into 8 plots. In each treatment, four plots are selected randomly. Cucumber variety is "Jin Chun 4". Fertilizer adopts hole application. The dosage of the bio-organic fertilizer is 30kg/mu.
Data are collected from the field at regular intervals. Statistics of morbidity and microfloras is conducted. After final harvesting, the difference of biomass among different treatments is calculated. The application of the microbial organic fertilizer formed from the re-fermentation of SQR-T037 and organic fertilizer can remarkably reduce the occurrence of wilt, lower morbidity by 33.1%, and increase cucumber yield by 1.3 folds (Table 5).
Microbial organic fertilizer was applied in the field with serious watermelon wilt in Tongxu, Henan in 2007.
Treatment of field experiment: 1. Apply organic fertilizer; 2. Apply the microbial organic fertilizer formed from the re-fermentation of SQR-T037 and organic fertilizer. Select a field with serious infection of wilt, and randomly divide the field into 8 plots, and randomly select four plots for each treatment. The variety of watermelon is 8424. Fertilizer adopts hole application. The dosage of the bio-organic fertilizer is 30kg/mu.
Data are collected from the field at regular intervals. Statistics on morbidity, disease index and microfloras is conducted. After final harvesting, the difference of biomass among different treatments is calculated. The application of the microbial organic fertilizer formed from the fermentation of SQR-T037 can remarkably reduce the occurrence of wilt. Comparing with the habitually applied fertilizers (non-grafted seedlings), morbidity is lowered by 20-40%, prevention and cure rate exceeds 95% and the yield of watermelon per mu increases by more than 600kg (Table 5).
The present invention proceeds from microflora, and develops a microbial organic fertilizer product that can remarkably eliminate the occurrence of the wilt of continuous cropping cucumber and watermelon and achieves above 85% of controlling rate (field experiment). The mechanism of this fertilizer product is that the functional bacteria and their active carbon and nitrogen sources provided by this fertilizer product provide a very good condition for the cultivation of beneficial microflora in the soil with continuous cropping obstacle, and enable the soil to quickly restore and establish the ecology and food chain of exogenous functional bacterial organisms; on the other hand, this fertilizer product has high content of organic nitrogen and organic phosphor. These nutritive substances are beneficial to the growth of the crops in the soil with continuous cropping obstacle and greatly improve the seedling standing rate and tolerance of cucumber and watermelon.

**Table 1 Chlorophyll content of laminae, activity of root system and dry weight of plants of the potted cucumber under different treatment**

| Treatment | Morbidity (%) | Chlorophyll content (SPAD) | Activity of root system (mg TTC $g^{-1}$ $h^{-1}$) | Dry weight of plants (g) |
|---|---|---|---|---|
| CK1 | 0 | 35.27±1.52 | 0.24±0.04 | 26.87±3.06 |
| CK2 | 98.9 | 27.93±0.69 | 0.11±0.02 | 12.77±5.77 |
| T1 | 93.3 | 30.07±1.17 | 0.14±0.04 | 16.70±3.06 |
| T2 | 25.6 | 35.27±3.00 | 0.33±0.04 | 30.86±0.65 |
| T3 | 4.4 | 39.67±0.94 | 0.40±0.05 | 34.83±2.57 |
| T4 | 0 | 46.80±1.68 | 0.60±0.03 | 36.61±1.26 |

**Table 2 Chlorophyll content of laminae, activity of root system and dry weight of plants of the potted watermelon under different treatment**

| Treat ment | Morbidity (%) | Chlorophyll content (SPAD) | Activity of root system (mg TTC $g^{-1}$ $h^{-1}$) | Dry weight of plants (g/cup) |
|---|---|---|---|---|
| CK1 | 0 | $63.38\pm1.98$ | $0.35\pm0.06$ | $1.32\pm0.02$ |
| CK2 | 95.38 | $56.01\pm1.18$ | $0.15\pm0.03$ | $0.68\pm0.01$ |
| T1 | 81.27. | $61.11\pm5.26$ | $0.21\pm0.04$ | $0.87\pm0.03$ |
| T2 | 2.60 | $92.28\pm2.23$ | $0.50\pm0.05$ | $3.03\pm0.35$ |

**Note**: The statistics of wilt morbidity follows international common rules: 0, the plant is healthy; 1, less than 10% of the plant wilts; 2, 11-20% of the plant wilts; 3, 21-50% of the plant wilts; 4, 50-100% of the plant wilts; 5, the plant dies. Calculation formula:

$$\text{Morbidity} = \frac{\sum (\text{Level} \times \text{Plant quantity})}{\text{Total plant quantity} \times \text{Top level}} \times 100\%$$

**Table 3 Difference of microbe content on root surface of the potted watermelon under different treatment**

| Treatment | Quantity of the microbes cucumber rhizosphere soil (cfu/g of soil) | | | | |
|---|---|---|---|---|---|
| | Pathogen | *Trichoderma* | Bacteria | Actinomyces | Fungi |
| CK$_1$ | $5.9\times10^2$ | $3.3\times10^3$ | $6.2\times10^6$ | $3.5\times10^4$ | $8.1\times10^3$ |
| CK$_2$ | $6.1\times10^4$ | $8.8\times10^3$ | $6.6\times10^6$ | $5.2\times10^4$ | $6.2\times10^4$ |
| T1 | $7.9\times10^4$ | $3.9\times10^3$ | $7.7\times10^6$ | $6.6\times10^4$ | $8.9\times10^4$ |
| T2 | $8.3\times10^3$ | $5.5\times10^5$ | $7.1\times10^6$ | $5.8\times10^4$ | $6.1\times10^5$ |
| T3 | $3.2\times10^3$ | $6.0\times10^5$ | $8.3\times10^6$ | $5.9\times10^4$ | $6.9\times10^5$ |
| T4 | $9.5\times10^2$ | $1.1\times10^6$ | $8.9\times10^6$ | $6.1\times10^4$ | $1.9\times10^6$ |
| CK$_1$ | $8.1\times10^2$ | $5.5\times10^3$ | $5.1\times10^6$ | $2.3\times10^4$ | $3.7\times10^3$ |
| CK$_2$ | $2.9\times10^4$ | $5.9\times10^3$ | $6.1\times10^6$ | $5.1\times10^4$ | $3.7\times10^4$ |
| T1 | $3.1\times10^4$ | $8.0\times10^3$ | $7.9\times10^6$ | $6.8\times10^4$ | $6.1\times10^4$ |
| T2 | $6.2\times10^3$ | $5.7\times10^5$ | $9.2\times10^6$ | $9.9\times10^4$ | $6.0\times10^5$ |

**Table 4 Changes of activity of the disease resistance-related enzymes at root under different treatment**

| Treatment | Activity of disease resistance-related enzymes at cucumber root | | |
|---|---|---|---|
| | PHENYLALANINE AMMONIA-LYASE ($\mu$g of cinnamic acid/g of fresh weight/portion) | CHITINASE ($\mu$g of N-acetyl-glucosamine/g of fresh weight/portion) | $\beta$-1,3-GLUCANASE ($\mu$g of glucose/g of fresh weight/portion) |
| CK1 | $16.20\pm1.47$ | $26.72\pm1.68$ | $106.39\pm6.55$ |
| CK2 | $36.00\pm5.88$ | $38.28\pm1.25$ | $153.26\pm9.44$ |
| T1 | $45.60\pm5.88$ | $39.89\pm4.46$ | $161.72\pm17.91$ |
| T2 | $78.60\pm3.43$ | $68.98\pm5.32$ | $332.29\pm9.35$ |
| T3 | $90.60\pm5.39$ | $87.18\pm4.41$ | $395.23\pm35.02$ |
| T4 | $112.20\pm9.31$ | $103.22\pm11.12$ | $615.08\pm50.58$ |
| CK1 | $29.00\pm6.21$ | $29.78\pm6.11$ | $51.22\pm3.32$ |

(continued)

| Treatment | Activity of disease resistance-related enzymes at cucumber root | | |
|---|---|---|---|
| | PHENYLALANINE AMMONIA-LYASE (μg of cinnamic acid/g of fresh weight/portion) | CHITINASE (μg of N-acetyl-glucosamine/g of fresh weight/portion) | β-1,3-GLUCANASE (μg of glucose/g of fresh weight/portion) |
| CK2 | 52.60±5.35 | 39.89±4.46 | 76.38±8.82 |
| T1 | 58.60±8.31 | 71.48±8.33 | 81.28±9.26 |
| T2 | 95.12±8.82 | 112.25±3.99 | 199.00±12.10 |

**Table 5 Effect of field experiment**

| Treatment | Effect of cucumber field experiment | | Effect of watermelon field experiment | |
|---|---|---|---|---|
| | Yield (kg/mu) | Morbidity (%) | Yield (kg/mu) | Morbidity (%) |
| Common composite fertilizer | 960.92 | 35.1 | 3402 | 28.1 |
| Bio-organic fertilizer | 1229.50 | 2.0 | 4096 | 4.50 |

**Claims**

1. A biocontrol strain SQR-T037 against the wilt of continuous cropping cucumber and watermelon, which belongs to *Trichoderma harzianum* and was collected by China General Micro biological Culture Collection Center (CGMCC) on September 22, 2009, having the culture collection number CGMCC No.3308, the main biological characteristics being as follows: the optimum growth temperature on potato-dextrose-agar culture medium is 30°C; the diameter of colony can reached 90mm and it will cover the whole dish and generate yellow pigment in the culture medium when incubated for 96 hours; the conidiophore is in a shape of bottle; the conidium is oval, 2.3-3.5μm long and 2.0-3.2μm wide, and its surface is smooth; at the beginning its color was yellow green, then it changed into dark green slowly.

2. A microbial organic fertilizer produced from the biocontrol strain according to claim 1.

3. The microbial organic fertilizer according to claim 2, wherein the content of biocontrol strain SQR-T037 is above $1 \times 10^8$ cfu/g, the content of total nitrogen is 4~5% (weight percent), above 90% of the total nitrogen is organic nitrogen, total nitrogen-phosphorus-potassium nutrient is 6~10% (weight percent) and organic matter is 30~35% (weight percent).

4. A method for producing the microbial organic fertilizer according to claim 2 or 3, comprising the following steps:

   1) The strain SQR-T037 according to claim 1 is incubated to potato-dextrose-agar (PDA) culture to conduct liquid fermentation production under the following conditions: the initial pH range is 6.5-7.2; the culture temperature is 25-30°C; DO (dissolved oxygen): ventilation range is 30~100% (v/v), 170rpm; the fermentation time is 48h; all mycelium pellets are broken into colony formation units of this strain in the late stage of fermentation, and the colony count of SQR-T037 in fermentation liquor is $\geq 1 \times 10^9$ cfu/mL;
   2) SQR-T037 fermentation liquor is respectively incubated to the mature livestock or poultry excrement compost and the mixture from microbial enzymatic hydrolysis of rapeseed meal at a dosage of 50L/t to conduct solid fermentation; the fermentation temperature is 30-50°C; during the fermentation, the material is turned over once a day; the fermentation is completed in 5-7 days, and in the end, a solid preparation of biocontrol strain SQR-T037 is obtained, wherein the colony count of this strain is above $1 \times 10^8$ cfu/g;
   3) The solid microbial agent SQR-T037 made from livestock or poultry excrement compost (50-80% v/v) and the solid microbial agent SQR-T037 made from the mixture from enzymatic hydrolysis of rapeseed meal (20-50% v/v) are thoroughly mixed; the mixture is ripened 2-3 days; during the ripening, it is turned over twice; the microbial organic fertilizer is evaporated at temperature of below 50°C till its water content is less than 30% and- after packaging - the microbial organic fertilizer that prevents and eliminates the wilt of continuous cropping cucumber and watermelon is obtained.

5. The method according to claim 4, wherein the potato-dextrose-agar culture used in step (1) is prepared by the following method (taking the preparation of 1L of culture medium for example):
Unpeel 200g of potato, cut it into small dice, and boil it in boiling water for 30min, then filter the mixture, add 20g of common sucrose into the filtrate, fix the volume at 1000 mL, adjust pH value to 6.5-7.0, and sterilize the liquor at 121°C for 20min.

6. The method according to claim 4 or 5, wherein the mature livestock or poultry excrement compost used in step (2) is **characterized in that**: the germination index of the mature livestock or poultry excrement compost is more than 98%, and the content of organic matter is ≥35%, as well as the content of organic nitrogen is 1.2-2% and water content is 25-30%.

7. The use of the microbial organic fertilizer according to claim 2 or 3 for the control of wilt on cucumber and watermelons.

**Patentansprüche**

1. Biokontrollstamm SQR-T037 gegen die Welke von kontinuierlich wachsender Gurke und Wassermelone, wobei der Stamm zu *Trichoderma harzianum* gehört und vom Chinesischen Allgemeinen Mikrobiologischen Kultur-Sammlungs-Center (CGMCC) am 22. September 2009 mit der Kultur-Sammlungs-Nummer CGMCC No. 3308 erfasst wurde, wobei die hauptsächlichen biologischen Eigenschaften die folgenden sind: die optimale Wachstumstemperatur auf Kartoffel-Dextrose-Agar-Kulturmedium ist 30°C; der Durchmesser der Kolonie kann 90 mm erreichen und sie wird die gesamte Petrischale bedecken und bildet ein gelbes Pigment im Kulturmedium, wenn für 96 Stunden inkubiert wird; die Konidiophore ist in Form einer Flasche; das Konidioma ist oval, 2,3 - 3,5 $\mu$m lang und 2,0 - 3,2 $\mu$m breit, und seine Oberfläche ist glatt; am Anfang ist seine Farbe gelb-grün, dann ändert es sich langsam in dunkelgrün.

2. Mikrobielles organisches Düngemittel hergestellt aus dem Biokontrollstamm gemäß Anspruch 1.

3. Mikrobielles organisches Düngemittel gemäß Anspruch 2, wobei der Gehalt an Biokontrollstamm SQR-T037 über 1x10$^8$ cfu/g ist, der Gesamtstickstoffgehalt ist 4 ~ 5% (Gewichtsprozent), über 90% des Gesamtstickstoffs ist organischer Stickstoff, Gesamt-Stickstoff-Phosphor-Kalium-Nährstoff ist 6 ~ 10% (Gewichtsprozent) und organische Substanz ist 30 ~ 35% (Gewichtsprozent).

4. Verfahren zur Herstellung des mikrobiellen organischen Düngemittels gemäß Anspruch 2 oder 3, umfassend die folgenden Schritte:

1) Der Stamm SQR-T037 gemäß Anspruch 1 wird auf Kartoffel-Dextrose-Agar (PDA) Kultur inkubiert, um Flüssigfermentationsproduktion unter den folgenden Bedingungen durchzuführen: der initiale pH-Bereich ist 6,5 - 7,2; die Kulturtemperatur ist 25 - 30°C; DO (gelöster Sauerstoff): Belüftungsbereich ist 30 ~ 100% (v/v), 170 rpm; die Fermentationszeit ist 48 Stunden; alle Mycelium-Pellets sind in einem späten Stadium der Fermentation in Kolonie bildende Einheiten dieses Stammes zerbrochen, und die Kolonieanzahl von SQR-T037 in der Fermentationsflüssigkeit ist ≥ 1x10$^9$ cfu/mL;
2) SQR-T037 Fermentationsflüssigkeit wird entsprechend mit reifem Vieh- oder Geflügel-Exkrement-Kompost und dem Gemisch aus mikrobieller enzymatischer Hydrolyse von Rapsschrot mit einer Dosierung von 50 L/t inkubiert, um Festfermentation durchzuführen; die Fermentationstemperatur ist 30 - 50°C; während der Fermentation wird das Material einmal am Tag umgedreht; die Fermentation ist nach 5 - 7 Tagen komplett, und am Ende wird eine feste Zubereitung des Biokontrollstammes SQR-T037 erhalten, wobei die Kolonieanzahl dieses Stammes über 1x10$^8$ cfu/g ist;
3) Der feste mikrobielle Wirkstoff SQR-T037, hergestellt aus Vieh- oder Geflügel-Exkrement-Kompost (50 - 80% v/v) und der feste mikrobielle Wirkstoff SQR-T037, hergestellt aus dem Gemisch der enzymatischen Hydrolyse von Rapsschrot (20 - 50 % v/v) wird sorgfältig gemischt; das Gemisch wird 2 - 3 Tage reifen gelassen; während des Reifens wird es zwei Mal umgedreht; das mikrobielle organische Düngemittel wird eingedampft bei einer Temperatur von unter 50°C bis dessen Wassergehalt geringer ist als 30% und - nach Verpackung - wird das mikrobielle organische Düngemittel, das die Welke von kontinuierlich wachsender Gurke und Wassermelone verhindert und eliminiert, erhalten.

5. Verfahren gemäß Anspruch 4, wobei die Kartoffel-Dextrose-Agar Kultur, die in Schritt 1) verwendet wurde, durch das folgende Verfahren (zum Beispiel die Herstellung von 1 L Kulturmedium) hergestellt wird:

Schälen von 200 g Kartoffeln, Schneiden dieser in kleine Würfel und Kochen im kochenden Wasser für 30 Minuten, dann Filtern des Gemisches, Zugabe von 20 g von gewöhnlicher Sucrose in das Filtrat, Einstellen des Volumens auf 1000 mL, Einstellen des pH-Wertes auf 6,5 - 7,0 und Sterilisieren der Flüssigkeit bei 121°C für 20 Minuten.

6. Verfahren gemäß Anspruch 4 oder 5, wobei der reife Vieh- oder Geflügel-Exkrement-Kompost, der in Schritt 2) verwendet wird, **dadurch gekennzeichnet ist, dass**: der Keimungsindex des reifen Vieh- oder Geflügel-Exkrement-Kompost höher als 98% ist und der Gehalt an organischem Material $\geq$ 35% ist, sowie der Gehalt an organischem Stickstoff 1,2 - 2% ist und der Wassergehalt 25 - 30% ist.

7. Verwendung des mikrobiellen organischen Düngemittels gemäß Anspruch 2 oder 3 zur Bekämpfung der Welke bei Gurken und Wassermelonen.

## Revendications

1. Souche SQR-T037 de biocontrôle contre le flétrissement de concombre et de pastèque en culture continue, qui appartient à *Trichoderma harzianum* et a été collectée par le China General Microbiological Culture Collection Center (CGMCC) le 22 septembre 2009, portant le numéro de collecte de culture CGMCC n°3308, les principales caractéristiques biologiques étant comme suit : la température de croissance optimale sur un milieu de culture de pomme de terre-dextrose-gélose est de 30°C ; le diamètre de colonie peut atteindre 90 mm et il couvrira l'ensemble de la boîte et générera un pigment jaune dans le milieu de culture lors d'une incubation de 96 heures ; le conidiophore se trouve sous la forme d'une bouteille ; la conidie est ovale, d'une longueur de 2,3-3,5 $\mu$m et d'une largeur de 2,0-3,2 $\mu$m, et sa surface est lisse ; au début sa couleur était jaune-vert, puis elle a viré lentement au vert foncé.

2. Fertilisant organique microbien produit par la souche de biocontrôle selon la revendication 1.

3. Fertilisant organique microbien selon la revendication 2, dans lequel la teneur en souche SQR-T037 de biocontrôle est supérieure à $1 \times 10^8$ ufc/g, la teneur en azote total est de 4-5% (% en poids), plus de 90% de l'azote total est constitué d'azote organique, le nutriment azote-phosphore-potassium total est de 6-10% (% en poids) et la matière organique est de 30-35% (% en poids).

4. Méthode de production du fertilisant organique microbien selon la revendication 2 ou 3, comprenant les étapes suivantes :

   1) la souche SQR-T037 selon la revendication 1 est incubée dans une culture pomme de terre-dextrose-gélose (PDA) afin de mener une production en fermentation liquide dans les conditions suivantes : la plage de pH initiale est de 6,5-7,2 ; la température de culture est de 25-30°C ; OD (oxygène dissous) : la plage de ventilation est de 30-100% (v/v), 170 tours/min ; la durée de fermentation est de 48 h ; tous les pellets de mycélium sont désintégrés en unités formant colonies de cette souche au dernier stade de fermentation, et le comptage de colonies de SQR-T037 dans la liqueur de fermentation est $\geq 1 \times 10^9$ ufc/ml ;
   2) la liqueur de fermentation de SQR-T037 est incubée respectivement dans un compost d'excréments de volailles ou de bétail mature et un mélange issu de l'hydrolyse enzymatique microbienne de tourteau de colza selon un dosage de 50 1/t afin de mener une fermentation solide ; la température de fermentation est de 30-50°C ; pendant la fermentation, le matériau est retourné une fois par jour ; la fermentation est terminée en 5-7 jours, et, à la fin, on obtient une préparation solide de souche SQR-T037 de biocontrôle, où le comptage de colonies de cette souche est supérieur à $1 \times 10^8$ ufc/g ;
   3) l'agent microbien solide SQR-T037 préparé à partir de compost d'excréments de volailles ou de bétail (50-80% v/v) et l'agent microbien solide SQR-T037 préparé à partir du mélange issu de l'hydrolyse enzymatique de tourteau de colza (20-50% v/v) sont mélangés soigneusement ; on fait mûrir le mélange pendant 2-3 jours ; pendant la maturation, il est retourné deux fois ; le fertilisant organique microbien est évaporé à une température inférieure à 50°C jusqu'à ce que sa teneur en eau soit inférieure à 30% et - après emballage - on obtient le fertilisant organique microbien qui empêche et élimine le flétrissement de concombre et de pastèque en culture continue.

5. Méthode selon la revendication 4, dans laquelle la culture pomme de terre-dextrose-gélose utilisée dans l'étape (1) est préparée par la méthode suivante (en prenant pour exemple la préparation de 1 1 de milieu de culture) :
   200 g de pommes de terre non pelées sont découpées en petits dés, et on les fait bouillir dans de l'eau bouillante pendant 30 minutes, puis on filtre le mélange, on ajoute 20 g de saccharose ordinaire au filtrat, on ajuste le volume à 1000 ml, on ajuste la valeur de pH à 6,5-7,0, et on stérilise la liqueur à 121°C pendant 20 min.

6. Méthode selon la revendication 4 ou 5, dans laquelle le compost d'excréments de volailles ou de bétail mature utilisé dans l'étape (2) est **caractérisé en ce que** l'indice de germination du compost d'excréments de volailles ou de bétail mature est supérieur à 98%, et la teneur en matière organique est $\geq$ 35%, ainsi que la teneur en azote organique est de 1,2-2% et la teneur en eau est de 25-30%.

7. Utilisation du fertilisant organique microbien selon la revendication 2 ou 3, pour le contrôle du flétrissement des concombres et des pastèques.

A

B

Fig.1

CK₁    T₄         T₃              CK₂   T₁    T₂

A

CK₁           CK₂            T₁              T₂

B

Fig. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 10105753 A

- CN ZL200610086

**Non-patent literature cited in the description**

- **CHO et al.** *BIOLOGICAL COONTROL OF FUSARIUM-OXYSPRORUM-F-SP-CUCUMBERING CAUSING CUCUMBER WILT BY GLIOCLADIUM-VIRENS AND TRICHODERMA-VIRENS*
- database. 1900000753

- **LIN WEI et al.** Test on the control efficacy of antagonistic microorganism on watermelon wilt disease. *JOURNAL OF GUANGXI AGRIC AND BIOL SCIENCE,* 31 December 2002, vol. 21 (4), 242-244